(19) Europäisches Patentamt
European Patent Office
Office européen des brevets

(11) **EP 3 490 634 B1**

(12) **EUROPÄISCHE PATENTSCHRIFT**

(45) Veröffentlichungstag und Bekanntmachung des Hinweises auf die Patenterteilung:
**30.04.2025 Patentblatt 2025/18**

(21) Anmeldenummer: **17764505.8**

(22) Anmeldetag: **20.07.2017**

(51) Internationale Patentklassifikation (IPC):
**A61M 3/02** (2006.01)    **A61B 1/12** (2006.01)
**F28F 3/12** (2006.01)    **F28F 27/02** (2006.01)

(52) Gemeinsame Patentklassifikation (CPC):
**A61M 3/022; A61B 1/128; A61M 3/02;**
**A61M 3/0201; F28F 3/12; F28F 27/02;**
A61M 2205/122; A61M 2205/127; A61M 2205/128;
A61M 2205/3334; A61M 2205/3368;
A61M 2205/3606; A61M 2205/366;
A61M 2205/6027; A61M 2206/14;    (Forts.)

(86) Internationale Anmeldenummer:
**PCT/DE2017/000215**

(87) Internationale Veröffentlichungsnummer:
**WO 2018/019317 (01.02.2018 Gazette 2018/05)**

(54) **VORRICHTUNG UND VERFAHREN ZUR DURCHFLUSSTEMPERIERUNG VON FLÜSSIGKEITEN BEI MEDIZINTECHNISCHEN VORRICHTUNGEN**

DEVICE AND METHOD FOR TEMPERING THE FLOW OF LIQUIDS IN MEDICAL DEVICES

DISPOSITIF ET PROCÉDÉ DE MISE EN TEMPÉRATURE DE LIQUIDES CIRCULANTS DANS DES DISPOSITIFS MÉDICO-TECHNIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.07.2016 DE 102016009173**

(43) Veröffentlichungstag der Anmeldung:
**05.06.2019 Patentblatt 2019/23**

(73) Patentinhaber: **W.O.M. World of Medicine GmbH 10587 Berlin (DE)**

(72) Erfinder:
• **LUTZ, Andreas**
  **12623 Berlin (DE)**
• **SCHÖNBORN, Karl-Heinz Günter**
  **12355 Berlin (DE)**

(74) Vertreter: **Gulde & Partner**
**Patent- und Rechtsanwaltskanzlei mbB**
**Berliner Freiheit 2**
**10785 Berlin (DE)**

(56) Entgegenhaltungen:
**DE-A1- 102010 036 295    US-A1- 2002 045 851**

EP 3 490 634 B1

(52) Gemeinsame Patentklassifikation (CPC): (Forts.)
F28D 2021/005; F28F 2250/06; F28F 2255/02

**Beschreibung**

Es ist bekannt, im Rahmen verschiedenster medizinischer Verfahren der Endoskopie, z. B. der Arthroskopie, der (Endo-)Urologie oder der Hysteroskopie, Körperhöhlen zu durchspülen. Hierbei wird üblicherweise mittels einer Pumpe, z. B. einer Rollendruckpumpe, eine Spülflüssigkeit in eine Körperhöhle gepumpt. Es hat sich dabei als vorteilhaft herausgestellt, die Spülflüssigkeit bei der Applikation an die Körpertemperatur anzugleichen, um - beispielsweise in der (Endo-)Urologie und der Hysteroskopie -eine lokale Hypothermie des Patienten-Körpers zu vermeiden. Im Stand der Technik sind hierzu verschiedene Lösungen bekannt (z. B. US 6,480,257, US 8,790,303, US 7,153,285, US 2003/0216689 A1), die jedoch unterschiedliche Nachteile aufweisen. Weitere Vorrichtungen aus dem Stand der Technik sind aus US 2002/0045851 A1 und DE 10 2010 036 295 A1 bekannt.

In der Arthroskopie kann es vorteilhaft sein, das Gelenk durch die Spülflüssigkeit zu kühlen, um entzündliche Prozesse zu verringern, Einblutungen zu vermeiden und/oder während der Operation die lokale Stoffwechselaktivität zu reduzieren.

Gegenstand der vorliegenden Erfindung ist eine neue Vorrichtung, die verschiedene Nachteile des Standes der Technik überwinden soll.

Gegenstand der vorliegenden Erfindung ist daher eine Vorrichtung zum Durchflusstemperieren medizinischer Spülflüssigkeiten gemäß Anspruch 1.

Vorteilhafte Ausgestaltungen der Erfindung werden nachfolgend erläutert und sind Gegenstand der Unteransprüche.

Die erfindungsgemäße Vorrichtung besteht daher aus einem Wärmetauschkörper, z.B. in Form einer Kassette, die von der zu temperierenden Flüssigkeit durchflossen wird und einem an die Form des Wärmetauschkörpers angepassten flächigen Temperierelementes.

In der bevorzugten Ausführungsform der Erfindung wird die zu temperierende Flüssigkeit von Raumtemperatur auf Körpertemperatur erwärmt, z.B. von 20°C auf 37°C. In diesem Fall ist das flächige Temperierelement als Flächenheizelement ausgestaltet.

In einer alternativen Ausführungsform der Erfindung wird die zu temperierende Flüssigkeit von Raumtemperatur heruntergekühlt, z.B. von 20°C auf 3-5°C. In diesem Fall ist das flächige Temperierelement als Flächenkühlelement ausgestaltet. Diese alternative Ausführungsform wird weiter unten ausführlich dargestellt.

Der Wärmetauschkörper kann unterschiedliche geometrische Formen aufweisen.

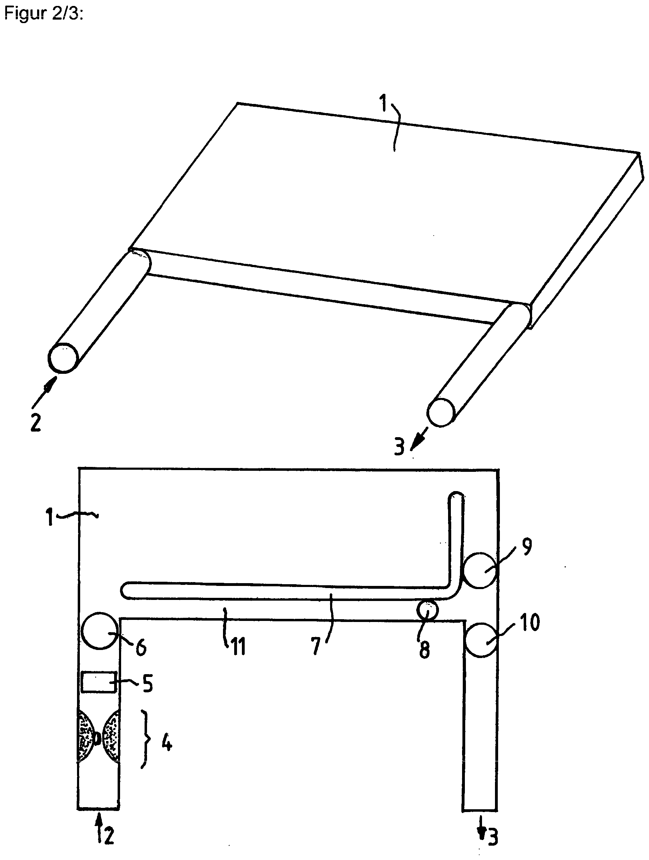

Wie in den anliegenden Figuren dargestellt, ist eine bevorzugte Ausführungsform die Form eines flachen Quaders. Das Innere des Quaders ist im Wesentlichen hohl und kann Spülflüssigkeit aufnehmen. Der Quader weist an einer Stelle einen Einlass, an einer anderen Stelle einen Auslass auf. Ein- und Auslass können, wie in Figur 2 dargestellt, an einer Quaderfläche angeordnet sein. Durch Zufluss von Flüssigkeit in den Einlass wird der Wärmetauschkörper mit der Flüssigkeit gefüllt, bis sie zum Auslass wieder austritt. Bevorzugt ist in Betriebsstellung der Auslass höher gelegen, als der Einlass, so dass die ursprünglich enthaltene Luft leichter entweichen kann. Alternativ kann der Wärmetauschkörper auch andere geometrische Formen aufweisen, wie z.B rund, oval, sechseckig, achteckig, usw..

Der Wärmetauschkörper ist vorzugsweise aus Kunststoff (z.B. Polycarbonat (PC), Polyethylenterephthalat (PET), Polyethylenterephthalat-Glycol (PETG), Polyvinylchlorid (PVC), Polypropylen (PP), Polyethylen (PE) oder Mischungen dieser Kunststoffe gefertigt. Dabei können die Kunststoffe auch keramikpulvergefüllt sein. Ferner besteht die Möglichkeit den Wärmetauschkörper aus Metall, insbesondere aus Metallfolien (z.B. Aluminiumfolie) zu fertigen. Die Einzelkomponenten des Wärmetauschkörpers können auch aus unterschiedlichen Materialien bestehen. Hierbei ist wenigstens eine Wärmetauschkörperwand so gefertigt, dass ein guter Wärmeübergang gewährleistet ist. Der Wärmeübergangskoeffizient dieser Wand (auch Wärmeleitwand genannt) sollte mindestens 1 W / (cm$^2$ K) betragen, vorzugsweise größer 2,5 W / (cm$^2$ K).

Als "Wärmeübergangskoeffizient a" bezeichnet man den Koeffizienten, der den Wärmetransport (P = $\Delta Q/\Delta t$) pro Flächeneinheit (P = $\Delta Q/\Delta t$) über eine Grenzfläche als Funktion der Temperaturdifferenz zwischen den beiden Seiten der Grenzfläche gemäß folgender Gleichung beschreibt:

$$P = \alpha \cdot \Delta A \cdot \Delta T$$

Hierzu sind generell alle wärmeleitenden Materialien geeignet. Bevorzugt wird hierzu eine Metallfolie, besonders bevorzugt eine Aluminiumfolie verwendet. Alternativ kann auch ein keramikpulvergefüllter Kunststoff herangezogen werden. Vorteilhafterweise werden die größten Flächen des Wärmetauschkörpers für den Wärmeübergang herangezogen, z. B. die Deckfläche oder Grundfläche eines flachen Quaders. In speziellen Ausführungsformen können auch mehrere Flächen derartig ausgestaltet sein, z. B. die Deckfläche und die Grundfläche eines quaderförmigen Wärmetauschkörpers.

Erfindungsgemäß enthält der Wärmetauschkörper zwei Strömungswege: Der Großteil des Wärmetauschkörpers wird durch das Heizelement aufgeheizt, während gleichzeitig die Möglichkeit eines "By-pass" gegeben ist, durch den Flüssigkeit direkt vom Ein- zum Ausgang fließen kann, ohne aufgeheizt zu werden. Der By-pass wird erfindungsgemäß durch entsprechende Ausformung des Wärmetauschkörperbodens innerhalb des Wärmetauschkörpers gebildet, wie es beispielhaft in Figur 2 gezeigt wird.

Alternativ kann in nicht beanspruchten Ausführungsformen der By-pass außerhalb des Wärmetauschkörpers gebildet werden, beispielsweise durch einen Schlauch welcher parallel zum Wärmetauschkörper verläuft. In jedem Fall dieser Ausführungsform sind Stellvorrichtungen, beispielsweise Ventile (z.B. in Form von Quetschventilen) vorgesehen, die den Flüssigkeitsstrom entweder in die Hauptkammer oder in den Bypass leiten. Bevorzugt läuft der By-pass innerhalb des Wärmetauschkörpers durch einen Bereich, der keinen Kontakt zum weiter unten beschriebenen Flächentemperierelement hat (siehe Figur 2). Diese Ausführungsform erlaubt es, die Ausgangstemperatur der Spülflüssigkeit schnell und effektiv zu regulieren, insbesondere bei Anwendungen, bei denen der Strömungsfluss sehr ungleichmäßig ist. Sollte hier, z.B. in Folge eines temporären Strömungsstillstands, die Fluidtemperatur in der Kammer zu hoch werden, kann über den Bypass gezielt ungeheizte Spülflüssigkeit zudosiert werden, um die Ausgangstemperatur konstant zu halten. Ferner kann diese verwendet werden, um eine ausreichend Regelungsgeschwindigkeit der Ausgangstemperatur zu erzielen, die auch bei starken Schwankungen der Durchflussrate eine sichere Temperatureinstellung garantiert.

Um den Flüssigkeitsstrom durch Wärmetauschkörper und By-Pass steuern zu können sind Teile des Wärmetauschkörpers aus elastischem Material (z.B. Silikon) gefertigt, um mittels in die Heizungsgehäuse integrierter Aktuatoren den Flüssigkeitsstrom durch Der Wärmetauschkörper bzw. den Bypass (nach Art eines Quetschventils) zu regeln. Je nach verwendetem Wandmaterial kann auch eine lokale Materialschwächung oder -formgebung (z.B. in der Art eines Faltenbalges) bereits ausreichen, um die nötige Flexibilität im Sinne einer reversiblen Verformbarkeit sicherzustellen. Vorzugsweise sind die Aktuatoren so eingestellt, dass im Ruhezustand, bei Ausfällen oder Störungen der Bypass durchströmt und die Heizstrecke nicht fluiddurchströmt ist. Auf diese Weise wird sichergestellt, dass das Fluid in keinem Fall dem Patienten in zu heißem Zustand zugeführt wird.

Der eingangs beschriebene Wärmetauschkörper wird erfindungsgemäß zusammen mit einem entsprechend angepassten Temperierelement, z.B. einem Heizelement verwendet. Das Heizelement ist so geformt, dass mindestens eine Wand des Wärmetauschkörpers flächig aufgeheizt werden kann. Derartige Heizelemente in Form einer flächigen Heizfolie werden kommerziell angeboten und bestehen aus einer zumeist einlagigen und flächig angeordneten ohmschen Heizwicklung, die in eine isolierende Kunststofffolie, beispielsweise aus Silikon oder Polyimid (Handelsname Kapton®), eingebettet ist.

Alternativ kann das Heizelement auch als Heizfläche mit einem drahtförmigen Heizleiter, der in Keramikpulver (= elektr. Isolation) verpresst ist, ausgestaltet sein, welcher seinerseits in eine metallische Außenhülse/Gehäuse verpresst ist.

In engen thermischen Kontakt mit dem Heizelement, z.B. der Heizfolie, können an einer oder mehreren Stellen Temperaturfühler angeordnet werden, um die Temperatur der Heizfolie messen und eine auf den Messungen basierende Temperaturregelung vornehmen zu können.

Als eine besondere Ausführungsform kann diese Heizfolie auch aus einem PTC-Kaltleiter gefertigt sein, der mit zunehmender Temperatur seinen elektrischen Widerstand erhöht. Auf diese Weise kann unter Umständen auf die Temperaturregelung verzichtet werden kann, weil der zunehmende Widerstand bei Temperaturerhöhung eine Abnahme der Heizleistung bewirkt. Derartige PTC-Kaltleiter und die dazugehörigen Regelungen sind Stand der Technik und bedürfen an dieser Stelle keiner weiteren Erläuterung.

Sinnvollerweise ist das Heizelement im Inneren eines Gehäuses angeordnet, welches eine praktisch beliebige äußere Form aufweisen kann. Entscheidend für den erfindungsgemäßen Einsatz ist eine Gehäuseöffnung zur Aufnahme des beschriebenen Wärmetauschkörpers, z.B. in Form eines Schlitzes. Die Öffnung ist selbstverständlich hinsichtlich der Geometrie so gestaltet, dass der Wärmetauschkörper in die Öffnung eingeführt werden kann. An einer Seite des durch die Öffnung erreichbaren Hohlraumes, z. B. der Unterseite, befindet sich das flächige Heizelement, z. B. in Form einer Heizfolie. Der oben beschriebene Wärmetauschkörper muss so in die Öffnung eingeführt werden, dass die Seite, die den Wärmeübergang gewährleistet, in Kontakt kommt mit dem Heizelement. Durch Einstellung der Heizleistung kann die Temperatur der Spülflüssigkeit geregelt werden.

Wie sich herausgestellt hat, wird der Wärmetauschkörper bevorzugt von unten beheizt. Aufgrund der thermischen Konvektion findet dabei eine thermische Durchmischung der Flüssigkeit im Wärmetauschkörper statt. Im Fall der weiter unter beschriebenen Betriebsform, bei der das Fluid gekühlt wird, ist das Kühlelement bevorzugt oben angeordnet. Da prinzipiell auch zwei Wände des Wärmetauschkörpers durch wärmeleitendes Material gebildet sein können, z.B. die Bodenfläche und die Deckfläche, kann auch von zwei Seiten beheizt oder gekühlt werden.

In einer weiteren Ausführungsform der Erfindung ist es möglich, sowohl eine Heizfläche, wie auch eine Kühlfläche im Gehäuse vorzusehen. Je nach Einstellung der Vorrichtung kann somit alternativ geheizt oder gekühlt werden.

Bevorzugt wird die Form der genannten Einführungsöffnung geometrisch so gestaltet, dass eine Fehlpositionierung des Wärmetauschkörpers weitgehend ausgeschlossen werden kann. Hierzu können ergänzende Symbole oder Farbkodierungen hilfreich sein.

Die erfindungsgemäße Vorrichtung kann wie folgt betrieben werden:

Aus einem Vorratsbehälter mit medizinischer Spülflüssigkeit führt ein Schlauch zum Eingang des Wärmetauschkörpers. Ein weiterer Schlauch verbindet den Ausgang des Wärmetauschkörpers mit dem Eingang einer medizinischen Pumpe, z. B. einer Rollenradpumpe. Vom Ausgang der Pumpe wird ein weiterer Schlauch zu einem medizinischen Instrument, wie zum Beispiel einem Endoskop. Alternativ kann das erfindungsgemäße Gerät auch ohne Pumpe in reiner Gravitationsförderung bertrieben werden; in diesem Fall führt der ausgangsseitigen Schlauch direkt vom Wärmetauschkörper zum Instrument, wobei alle übrigen erfindungsgemäßen Kennzeichen unverändert bleiben. Der Wärmetauschkörper wird in die Heizvorrichtung eingeführt. Die durch die erfindungsgemäße Heizvorrichtung auf die gewünschte Temperatur (z. B. Körpertemperatur des Patienten) vorgewärmte Flüssigkeit wird mittels der Spülpumpe in den Körper des Patienten geführt.

Nachfolgend werden bevorzugte Ausführungsformen der Erfindung beschrieben:
Der Wärmetauschkörper ist vorzugsweise als Einwegartikel konzipiert und wird daher nach der einmaligen Benutzung entsorgt. Der Wärmetauschkörper besteht vorzugsweise aus Kunststoff (z. B. Polycarbonat (PC), Polyethylenterephthalat (PET), Polyethylenterephthalat-Glycol (PETG), Polyvinylchlorid (PVC), Polypropylen (PP), Polyethylen (PE) oder Mischungen dieser Kunststoffe), welcher durch Tiefziehen ausgeformt ist. Alternativ kommen natürlich auch andere Techniken in Betracht, wie z.B. Spritzguss oder 3D-Druck. Auf diese Weise kann die Deckseite der Wärmetauschkörper in einfacher Weise durch eine aufgeklebte oder aufgeschweißte Aluminiumfolie (Stärke z. B. 20-90 Mikrometer, bevorzugt 60 Mikrometer) überzogen werden. In einer bevorzugten Ausführungsform ist die Aluminiumfolie auf der Seite, die die Innenseite des Wärmetauschkörpers bildet mit einer Kunststoffschicht (z. B. 1-5 Mikrometer, bevorzugt 2 Mikrometer Polypropylen) überzogen. Auf diese Weise wird ein direkter Kontakt des Fluides mit Aluminium verhindert, ohne den notwendigen Wärmeübergang zu stören.

Um die nötige Heizleistung übertragen zu können beträgt die Grundfläche des Wärmetauschkörpers im Allgemeinen zwischen 100 und 1.600cm$^2$ (z.B. 10 mal 10 cm bis 40 mal 40 cm).

Die Schlauchanschlüsse sind beispielsweise Spritzgussteile oder sonstige Kunststoffformteile und werden vorzugsweise aus demselben Kunststoff gefertigt, wie das Grundteil des Wärmetauschkörpers. Die Schlauchanschlüsse werden jeweils in entsprechend vorbereitete Öffnungen gesteckt und mit dem Wärmetauschkörper verschweißt oder verklebt. Alternativ können die Schlauchanschlüsse auch durch Tiefziehen, Extrusion oder 3D-Druck direkt angeformt werden.

Der Wärmetauschkörper ist vorzugsweise flach mit einer Höhe von 5-20 mm. Die Gehäuseöffnung ist entsprechend darauf angepasst ausgestaltet. Vorzugsweise ist die Gehäuseöffnung so dimensioniert, dass das Bedienungspersonal nicht mit den Fingern in die Öffnung geraten kann, um Unfälle durch Verbrennungen zu vermeiden.

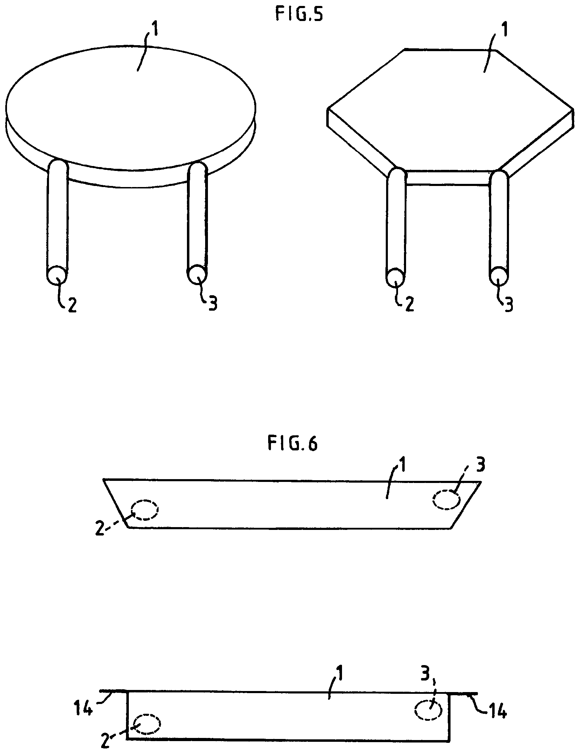

Weiterhin kann durch entsprechende Formgebung von Wärmetauschkörper und Gehäuseöffnung sichergestellt werden, dass der Wärmetauschkörper in der richtigen Art und Weise in das Gehäuse eingelegt wird, z.B. durch leichte trapezförmige Abweichung von der Quaderform (Figur 6 oben) mit entsprechender Formung der Gehäuseöffnung oder durch Anformung von Führungsleisten an der Wärmetauschkörperseite (Figur 6 unten) mit entsprechenden Führungsschlitzen im Gehäuse. Alternativ oder ergänzend können auf Wärmetauschkörper und Gehäuse angebrachte Symbole oder Farbkodierungen die Fehlbedienung ausschließen.

Weiterhin vorzugsweise enthält die Temperiervorrichtung einen Temperatursensor zur Kontrolle der Ausgangstemperatur der Spülflüssigkeit. Hierzu kann beispielsweise ein Temperatursensor in den Ausgang des Wärmetauschkörpers integriert sein.

Wie sich herausgestellt hat, ist bei Verwendung einer Aluminiumfolie für den Wärmeübergang die Wärmeleitung dieser Folie so gut, dass die Temperaturmessung durch ein Kontaktthermometer, welche nahe dem Ausgang des Wärmetauschkörpers positioniert ist, ausreichend, um die Temperatur präzise genug messen zu können. Auf diese Weise kann der Temperatursensor Bestandteil des Gehäuses sein und kann wiederverwendet werden.

In einer Weiterentwicklung der beschriebenen Erfindung weist das Gehäuse eine Unterdruckpumpe auf. Das Erzeugen eines Unterdrucks auf der Seite des Wärmeübergangs ermöglicht ein Anpressen des Wärmetauschkörpers an das Temperierelement, so dass der Wärmeübergang verbessert wird. Der oder die Ansaugstutzen der Pumpe wird im Bereich des Heizelementes liegen. Es wird sich empfehlen, wenn um das flächige Heizelement herum ein Dichtungsmaterial (z. B. eine Silikondichtung in Form einer umlaufenden Dichtschnur) angeordnet ist, damit der Unterdruck leicht gehalten werden kann. Bei dieser Ausführungsform der Erfindung wird ein Druck zwischen Wärmetauschkörper und Heiz- bzw. Kühlfläche von 0,3-0,05 bar angestrebt, bevorzugt ca. 0,1 bar. Bei diesem Druck wird die wärmeleitende Wand des Wärmetauschkörpers (z.B. die Aluminiumfolie) auf weniger als 1 $\mu$m an die Heiz- bzw. Kühlfläche herangezogen. Gleichzeitig ist bei einem Druck von 0,3-0,05 bar eine hervorragender Wärmetransport gegeben.

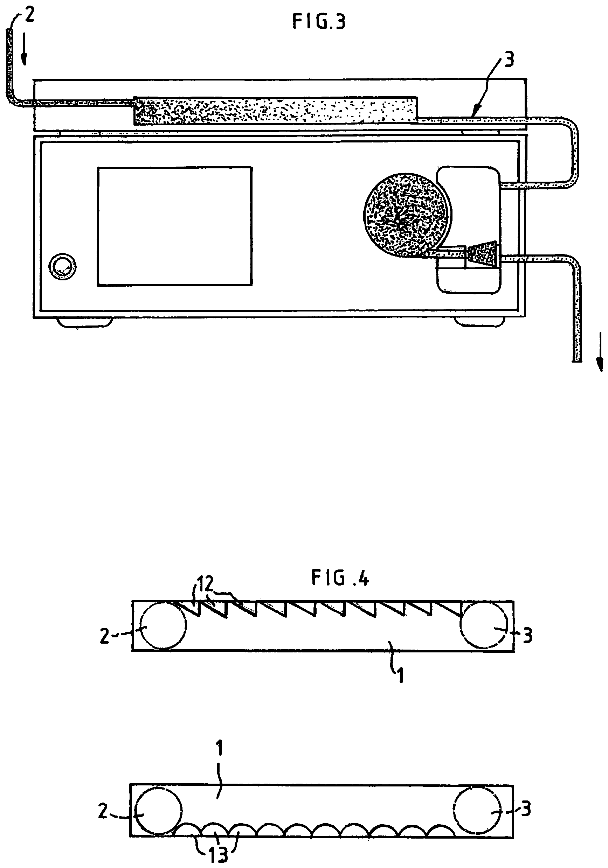

In einer weiteren Ausführungsform der Erfindung enthalten eine oder mehrere Innenseiten der Wärmetauschkörperinnenwände eine Strukturierung, z. B. in Form einer wellenförmigen, sägezahnartigen oder fischgrätartig strukturierten Ausgestaltung der Innenflächen (Figur 4). Es hat sich gezeigt, dass eine derartige Strukturierung zu einer Verwirbelung der durchgeleiteten Flüssigkeit und somit zu einer thermischen Homogenisierung der Strömung und der Temperatur des Wärmetauschkörpers beiträgt. Eine Strukturierung derjenigen Innenwand, die der Wand mit Wärmeübergang gegenüberliegt hat sich als besonders vorteilhaft erwiesen.

Ja nach Größe der Wärmetauschkörper und Elastizität des Wärmetauschkörpermaterials können im Inneren auch Stützelemente angebracht sein (z.B. in Zylinderform) um ein Kollabieren der Wärmetauschkörper zu vermeiden. Die Wärmetauschkörperwände können auch Versteifungsvorrichtungen (z.B. Rippen) enthalten.

Es wird sich empfehlen, dass das flächige Heizelement der Heizvorrichtung eine Maximaltemperatur erreicht, die etwas über der Körpertemperatur von Patienten liegt (z.B. 39°C). Zur Sicherheit wird die Temperatur des flächigen Heizelementes durch entsprechende Temperatursensoren überwacht werden. Weiterhin wird die Ausgangstemperatur der Spülflüssigkeit aus dem Wärmetauschkörper durch einen Temperatursensor überwacht. Dieser kann, wie oben dargestellt, Bestandteil des Wärmetauschkörpers sein. Aus Kostengründen würde sich empfehlen, den Temperatursensor zum Bestandteil der Heizvorrichtung zu machen, z. B. in Form eines Kontaktsensors, der die Temperatur der Oberfläche der Aluminiumfolie misst.

Es hat sich herausgestellt, dass es zur Optimierung der Temperierregelung vorteilhaft ist, wenn der Fluss durch den Wärmetauschkörper gemessen wird. Wenn die erfindungsgemäße Vorrichtung in Kombination mit einer Pumpe z.B. einer medizinischen Rollenradpumpe, betrieben wird, dann ist es im Allgemeinen ausreichend, die Flussrate der Pumpe zu bestimmen. Wenn die Flussrate nicht anderweitig bestimmt wird und zur Verfügung steht, kann die erfindungsgemäße Vorrichtung auch zur gleichzeitigen Durchflussmessung hergerichtet sein. Die Flussmessung kann beispielsweise nach dem Venturi-Prinzip erfolgen. Hierzu muss der Flussquerschnitt, beispielsweise im Bereich des Flüssigkeitseingang- oder des Flüssigkeitsausgangs verengt werden. Durch Messung des Differenzdruckes (in Verengung und nach Verengung) kann der Durchfluss bestimmt werden. Zur Messung des Druckes sind entsprechende Sensoren erforderlich. Die Drucksensoren können dabei direkt in den Wärmetauschkörper, d.h. in dessen Ein- bzw. Ausgang, integriert sein. Alternativ kann an den Messstellen eine flexible Membran angeordnet sein, deren Auslenkung durch entsprechende Druckaufnehmer im Gehäuse zur Druckmessung herangezogen wird.

Allernativ kann die Flussrate auch thermisch gemessen werden: Hierzu muss an geeigneter Stelle des Wärmetauschkörpers, z.B. im Bereich des Flüssigkeitsein- bzw. Flüssigkeitsausganges ein Temperatursensor angeordnet sein. In unmittelbarer Nähe des Temperatursensors muss ein Heizelement, z.B. eine Wiederstandheizung angeordnet sein. Das Heizelement kann kontinuierlich oder diskontinuierlich heizen, wobei die Heizleistung bestimmt wird. Die erzielte Temperaturerhöhung in der Umgebung des Heizelementes, welche durch den Temperatursensor gemessen wird, ist ein Maß für die Flussrate. Bei hoher Flussrate wird sich keine nennenswerte Temperaturerhöhung einstellen, während bei geringer Flussrate eine Temperaturerhöhung über das Thermometer gemessen wird. Der Vorteil dieser Art der Flussmessung besteht darin, dass sowohl das Heizelement, wie auch der Temperatursensor nicht Bestandteil des Wärmetauschkörpers sein müssen, sondern durch Kontakt zur Oberfläche des Wärmetauschkörpers die Messung bewirken können. Auf diese Weise kann der Wärmetauschkörper in einfachster und damit preiswertester Weise hergestellt werden.

Die erfindungsgemäße Vorrichtung ist in der Lage bis zu 800 ml/min wässrige Spülflüssigkeit von Raumtemperatur (20°C) auf 38°C aufzuheizen.

Die erfindungsgemäße Vorrichtung weist eine Reihe von Vorteilen gegenüber dem Stand der Technik auf. Zunächst ist die erfindungsgemäße Vorrichtung einfach in das bestehende medizintechnische Equipment zu integrieren. Die Vorrichtung kann ergänzend zu bestehenden Pumpsystemen (z.B. einer Rollenradpumpe) verwendet werden, welche in üblicher Weise weiterbetrieben werden kann. Alternativ kann die erfindungsgemäße Vorrichtung auch als "*stand-alone*"-Lösung verwendet werden. In diesem Fall fließt die Flüssigkeit aus einem erhöht angebrachten Vorratsbehälter allein durch Schwerkraftwirkung ("Gravitationsförderung") durch den Wärmetauschkörper zum medizinischen Instrument, z.B. Endoskop.

Durch die Verwendung der Einmalwärmetauschkörper kann die notwendige Sicherheit, insbesondere die Sterilität in einfacher Weise gewährleistet werden. Die Herstellungskosten der Wärmetauschkörper sind dabei relativ gering.

Weiterhin wird durch die erfindungsgemäße Vorrichtung nur derjenige Teil der Spülflüssigkeit erwärmt, der auch unmittelbar zum Verbrauch ansteht. Die erfindungsgemäße Vorrichtung vermeidet dadurch, dass beispielsweise der gesamte Spülflüssigkeitsvorratsbehälter erwärmt werden muss, was sich negativ auf die Stabilität der Lösung auswirken würde.

Insbesondere die Ausführungsform mit integriertem Bypass ermöglicht auch ein hochpräzises Regulieren der Flüssigkeitstemperatur auch bei stark alternierendem Flüssigkeitsstrom.

Wie oben bereits erwähnt, kann die erfindungsgemäße Vorrichtung auch zur Durchflusskühlung herangezogen werden. Derartige Kühlungen sind beispielsweise für den Einsatz in der Arthroskopie vorteilhaft, um Schwellungen, Blutungen und das Schmerzempfinden des Patienten zu minimieren. Hierzu kann die Spülflüssigkeit auf wenige Grad über den Gefrierpunkt (z.B. 1-10°C, bevorzugt 2-5°C) herunter gekühlt werden. In dieser Ausführungsform der Erfindung wird statt eines flächigen Heizelementes ein flächiges Kühlelement verwendet. Es kann sich hierbei um einen flächig ausgeformten kompressorbetriebenen Kühler handeln. Alternativ kann auch ein Kältemittel zur Kühlung herangezogen werden. In einer weiteren Ausführungsform der Erfindung kann auch ein Peltier-Element verwendet werden. In der Ausführungsform mit einem derartigen Peltier-Element kann durch Stromflussumkehr (Umpolen) sowohl eine Kühlung als auch eine Heizung ermöglicht werden.

Wie weiter oben bereits erwähnt, findet die Kühlung des Wärmetauschkörpers bevorzugt von oben statt. Selbstverständlich ist es auch möglich mehrere Seiten, z.B. Grundfläche und Deckfläche eines quaderförmigen Wärmetauschkörpers zu kühlen. Die weiteren oben geschilderten alternativen Ausführungsformen der Erfindung, können in der Ausführungsform mit Kühlung völlig analog verwendet werden, wie beispielsweise die Temperaturmessung und oder die Flussmessung. Auch die Ausführung eines "By-Pass" ist analoger Weise möglich, um im Bedarfsfall Spülflüssigkeit mit Raumtemperatur zuzumischen.

In einer weiteren möglichen Ausführungsform der Erfindung kann der Wärmetauschkörper auch von zwei unterschiedlichen Seiten (z.B. Grundfläche und Deckfläche) beheizt und gekühlt werden. So ist es beispielsweise denkbar das Gehäuse auf einer Seite (z.B. der Unterseite) mit einem Heizelement auszustatten und auf einer anderen Seite (z.B. der Oberseite) mit einem Kühlelement. Bei Verwendung eines Wärmetauschkörpers mit zwei wärmeleitenden Wänden, kann je nach Betriebsart eine Kühlung oder eine Heizung erfolgen. Unter Umständen kann es sogar zweckmäßig sein, ein erfindungsgemäßes Gerät mit der Fähigkeit zu Kühlung und Heizung zu betreiben, um die Fluidtemperatur schneller, effektiver und/oder präzise regeln zu können.

## Erläuterung der Figuren

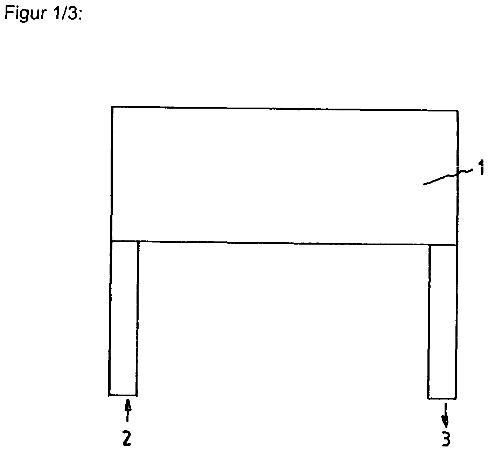

**Figur 1** zeigt die Grundform eines erfindungsgemäßen Wärmetauschkörpers (1) in der Draufsicht (oben) und perspektivisch (unten). Das Wärmetauschkörpergehäuse ist dabei im Wesentlichen quaderförmig, wobei zwei Anschlüsse als Ein- (2) bzw. Ausfluss (3) dargestellt sind.

**Figur 2** zeigt eine spezielle Ausführungsform des Wärmetauschkörpers (1) mit einem Bypass (11). Der By-pass wird innerhalb des Körpers durch eine Trennwand (7) vom Rest des Volumens abgetrennt. Der Wärmetauschkörper weist an mindestens zwei Stellen verformbare Elemente (8, 9) auf, die nach Art eines Quetschventils die Strömung durch den Hauptteil bzw. den Bypass regeln. Zusätzlich sind Temperatursensoren vorgesehen, die die Temperatur des Eingangs (6) bzw. des Ausgangs (10) messen. Ferner wird ein Durchflussmesser nach dem Venturi-Prinzip dargestellt: Hierzu ist im Zufluss eine Verengung ausgebildet (4). Drucksensoren in bzw. nach (5) der Engstelle erlauben durch Bestimmung des Differenzdruckes die Berechnung des Flüssigkeitsstroms.

**Figur 3** zeigt schematisch die Benutzung der erfindungsgemäßen Vorrichtung:

Das Flüssigkeitsreservoir wird mittels eines Schlauches in den Wärmetauschkörper geführt, welche sich in dem Gehäuse mit der Flächenheizvorrichtung befindet. Am Ausgang des Wärmetauschkörpers ist ein weiterer Schlauch angeschlossen, der zur Rollenradpumpe führt. Am Ausgang der Rollenradpumpe befinden sich die zum Patienten führenden Instrumente. Die Figur zeigt deutlich, dass sich die erfindungsgemäße Vorrichtung in einfacher Weise als Ergänzung zum vorhandenen medizinischen Equipment eignet.

**Figur 4** zeigt einen Querschnitt eines Wärmtauschkörpers mit sägezahnförmiger Ausgestaltung der Deckfläche (oben) bzw. wellenförmiger Ausgestaltung des Bodens (unten) Die Strömung durch einen derartigen Wärmetauschkörper führt zu einer Verwirbelung der Flüssigkeit mit der Folge eines besseren Wärmeübergangs.

**Figur 5** zeigt einen Querschnitt eines Wärmtauschkörpers mit trapezförmiger Form (oben) bzw. angeformten Führungsleisten (14) (unten). In Kombination mit der komplementär ausgestalteten Gehäuseöffnung können fehlerhafte Einführungen zuverlässig vermieden werden.

**Figur 6** zeigt Wärmetauschkörper mit anderer Geometrie, wie z.B. rund (oben) oder sechseckig (unten).

## Patentansprüche

**1.** Vorrichtung zum Durchflusstemperieren medizinischer Spülflüssigkeiten, enthaltend

einen Wärmetauschkörper (1) mit mindestens einem Ein- und Ausgang (6, 10), mit jeweiligem Schlauchanschluss, wobei der Wärmetauschkörper durch seine Wärmetauschkörperwände einen geschlossenen Hohlraum bildet wobei mindestens eine Wärmetauschkörperwand einen Wärmeübergangskoeffizienten größer als 1 W / (cm$^2$ K) aufweist, wobei der Wärmetauschkörper zwei Strömungswege aufweist, wobei die zwei Strömungswege im Inneren des Wärmetauschkörpers durch Ausformung des Wärmetauschkörperbodens gebildet werden, wobei ein Strömungsweg keinen thermischen Kontakt zum Temperierelement aufweist, wobei die Wärmetauschkörperwände zumindest stellenweise reversibel verformbar sind, so dass je nach Verformung die Strömungswege steuerbar sind,

wobei die Vorrichtung einen Temperatursensor zur Kontrolle der Ausgangstemperatur der Spülflüssigkeit enthält,

wobei über den Strömungsweg, der keinen thermischen Kontakt zum Temperierelement aufweist, gezielt untemperierte Spülflüssigkeit zudosiert werden kann, um die Temperatur am Wärmetauschkörperausgang konstant zu halten,

die Vorrichtung ferner enthaltend mindestens ein Flächentemperierelement passend zum Wärmetauschkörper,

wobei das Flächentemperierelement in ein Gehäuse mit einer Öffnung integriert ist,

wobei die Wärmetauschkörperwand mit einem Wärmeübergangskoeffizienten größer als $1\,W/(cm^2\,K)$ in Betriebsstellung Kontakt zum Flächentemperierelement hat.

2. Vorrichtung gemäß Anspruch 1, **gekennzeichnet durch** eine im Wesentlichen quaderförmige Form des Wärmetauschkörpers.

3. Vorrichtung gemäß Anspruch 1, **dadurch gekennzeichnet, dass** die den Wärmeübergang gewährleistende Wand durch eine Aluminiumfolie gebildet wird.

4. Vorrichtung gemäß Anspruch 1, **dadurch gekennzeichnet, dass** das flächige Temperierelement durch eine Heizfolie gebildet wird.

5. Vorrichtung gemäß Anspruch 1, **dadurch gekennzeichnet, dass** das Gehäuse mindestens ein Kontaktthermometer aufweist, welches in Betriebsstellung der Vorrichtung auf der Wand aufliegt, welche den Wärmeübergang gewährleistet.

6. Vorrichtung gemäß Anspruch 1, **dadurch gekennzeichnet, dass** das Gehäuse eine Vakuumpumpe aufweist, die einen Unterdruck zwischen Wärmetauschkörperwand und Temperierelement erzeugt.

7. Vorrichtung gemäß Anspruch 6, **dadurch gekennzeichnet, dass** der genannte Unterdruck zwischen Wärmetauschkörperwand und Temperierelement 0,05 bis 0,3 bar, bevorzugt 0,1 bar beträgt.

8. Vorrichtung gemäß Anspruch 1, **dadurch gekennzeichnet, dass** mindestens eine Wärmetauschkörperwand eine oberflächliche Strukturierung aufweist.

9. Vorrichtung gemäß Anspruch 1, **dadurch gekennzeichnet, dass** der Wärmetauschkörper im Bereich des Ein- und/oder Ausgangs eine Verengung des Strömungsquerschnittes aufweist, wobei in der Verengung sowie vor und/oder nach der Verengung jeweils eine Messstelle zur Druckmessung eingerichtet ist.

10. Vorrichtung gemäß Anspruch 1, **dadurch gekennzeichnet, dass** das Gehäuse im Bereich des Ein- und/oder Ausgangs des in Betriebsstellung befindlichen Wärmetauschkörpers mindestens einen Sensor zur thermischen Flussmessung aufweist, wobei der Sensor zur thermischen Flussmessung ein Heizelement und einen Temperatursensor enthält.

## Claims

1. A device for tempering the flow of medical irrigation liquids,

   including a heat exchange body (1) with at least one inlet and outlet (6, 10) each having a tube port,
   wherein the heat exchange body forms a closed cavity through its heat exchange body walls,
   wherein at least one heat exchange body wall has a heat transfer coefficient greater than $1W/(cm^2\,K)$,
   wherein the heat exchange body has two current paths,
   wherein the two current paths are formed on the inside of the heat exchange body through shaping of the heat exchange body base,
   wherein one current path has no thermal contact with the tempering element,
   wherein the heat exchange body walls are reversibly shaped at least in places so that the current paths are controllable depending on the shape,
   wherein the device includes a temperature sensor for controlling the outlet temperature of the irrigation liquid,
   wherein, via the current path that has no thermal contact with the tempering element, untempered irrigation liquid can be added in a targeted manner in order to maintain a constant temperature at the heat exchange body outlet,
   the device further including at least one surface tempering element which matches the heat exchange body,
   wherein the surface tempering element is integrated into a housing with an opening,
   wherein the heat exchange body wall with a heat transfer coefficient greater than $1W/(cm^2\,K)$ is in contact with the surface tempering element in the operating position.

2. The device according to Claim 1, **characterized by** a substantially parallelepiped shape of the heat exchange body.

**3.** The device according to Claim 1, **characterized in that** the wall providing the heat transfer is formed by an aluminum foil.

**4.** The device according to Claim 1, **characterized in that** the surface tempering element is formed by a heating foil.

**5.** The device according to Claim 1, **characterized in that** the housing has at least one contact thermometer which, in the operating position of the device, rests on the wall providing the heat transfer.

**6.** The device according to Claim 1, **characterized in that** the housing has a vacuum pump which generates an underpressure between the heat exchange body wall and the tempering element.

**7.** The device according to Claim 6, **characterized in that** said underpressure between the heat exchange body wall and the tempering element is 0.05 to 0.3 bar, preferably 0.1 bar.

**8.** The device according to Claim 1, **characterized in that** at least one heat exchange body wall has superficial structuring.

**9.** The device according to Claim 1, **characterized in that** the heat exchange body has a constriction of the current cross-section in the area of the inlet and/or outlet, wherein one measuring location for pressure measurement is set up in each of the constriction as well as before and/or after the constriction.

**10.** The device according to Claim 1, **characterized in that** the housing has at least one sensor for thermal flow measurement in the area of the inlet and/or outlet of the heat exchange body in the operating position, wherein the sensor for thermal flow measurement includes a heating element and a temperature sensor.

**Revendications**

**1.** Dispositif de thermorégulation en flux de liquides de rinçage médicaux, contenant

un corps d'échange de chaleur (1) avec au moins une entrée et une sortie (6, 10), avec un raccord de tuyau respectif, le corps d'échange de chaleur formant une cavité fermée par ses parois de corps d'échange de chaleur, au moins une paroi de corps d'échange de chaleur présentant un coefficient de transfert de chaleur supérieur à 1 W/(cm$^2$K), le corps d'échange de chaleur présentant deux voies d'écoulement, les deux voies d'écoulement étant formées à l'intérieur du corps d'échange de chaleur par moulage du fond du corps d'échange de chaleur, une voie d'écoulement ne présentant pas de contact thermique avec l'élément de thermorégulation, les parois de corps d'échange de chaleur étant déformables de manière réversible au moins par endroits, de telle sorte que les voies d'écoulement peuvent être commandées en fonction de la déformation, le dispositif contenant un capteur de température pour contrôler la température de sortie du liquide de rinçage, du liquide de rinçage non thermorégulé pouvant être ajouté de manière ciblée par le biais de la voie d'écoulement qui ne présente pas de contact thermique avec l'élément de thermorégulation, afin de maintenir constante la température à la sortie du corps d'échange de chaleur, le dispositif contenant en outre au moins un élément de thermorégulation de surface adapté au corps d'échange de chaleur, l'élément de thermorégulation de surface étant intégré dans un boîtier avec une ouverture, la paroi de corps d'échange de chaleur qui a un coefficient de transfert de chaleur supérieur à 1 W/(cm$^2$K) étant en contact avec l'élément de thermorégulation de surface en position de fonctionnement.

**2.** Dispositif selon la revendication 1, **caractérisé par** une forme essentiellement parallélépipédique du corps d'échange de chaleur.

**3.** Dispositif selon la revendication 1, **caractérisé en ce que** la paroi assurant le transfert de chaleur est formée par une feuille d'aluminium.

**4.** Dispositif selon la revendication 1, **caractérisé en ce que** l'élément de thermorégulation plat est formé par une feuille chauffante.

**5.** Dispositif selon la revendication 1, **caractérisé en ce que** le boîtier présente au moins un thermomètre de contact qui, en position de fonctionnement du dispositif, repose sur la paroi assurant le transfert de chaleur.

**6.** Dispositif selon la revendication 1, **caractérisé en ce que** le boîtier présente une pompe à vide qui crée une dépression entre la paroi de corps d'échange de chaleur et l'élément de thermorégulation.

**7.** Dispositif selon la revendication 6, **caractérisé en ce que** ladite dépression entre la paroi de corps d'échange de chaleur et l'élément de thermorégulation est de 0,05 à 0,3 bar, de préférence 0,1 bar.

8. Dispositif selon la revendication 1, **caractérisé en ce qu'**au moins une paroi de corps d'échange de chaleur présente une structuration superficielle.

9. Dispositif selon la revendication 1, **caractérisé en ce que** le corps d'échange de chaleur présente un rétrécissement de la section transversale d'écoulement dans la zone de l'entrée et/ou de la sortie, un point de mesure étant aménagé dans le rétrécissement ainsi qu'avant et/ou après le rétrécissement pour mesurer la pression.

10. Dispositif selon la revendication 1, **caractérisé en ce que** le boîtier présente, dans la zone de l'entrée et/ou de la sortie du corps d'échange de chaleur se trouvant en position de fonctionnement, au moins un capteur pour la mesure du flux thermique, le capteur pour la mesure du flux thermique contenant un élément chauffant et un capteur de température.

Figur 1/3:

Figur 2/3:

FIG.3

FIG.4

FIG.5

FIG.6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6480257 B
- US 8790303 B
- US 7153285 B
- US 20030216689 A1
- US 20020045851 A1
- DE 102010036295 A1